## Europäisches Patentamt
## European Patent Office
## Office européen des brevets

(19)

(11) Veröffentlichungsnummer: **0 063 815**
**B1**

(12) **EUROPÄISCHE PATENTSCHRIFT**

(45) Veröffentlichungstag der Patentschrift:
02.01.85

(21) Anmeldenummer: 82103533.4

(22) Anmeldetag: 27.04.82

(51) Int. Cl.³: **C 07 C 43/04**, C 07 C 41/06,
C 07 C 41/42, C 07 C 31/12,
C 07 C 31/10, C 07 C 29/04,
C 07 C 29/86 // C07C31/04,
C07C11/06, C07C11/08,
C07C11/09, C07C11/167,
C07C9/08, C07C9/10, C07C9/12,
C07C7/04, C07C5/13,
C07C5/333, C07C5/22,
C07C5/25

(54) Verfahren zur Herstellung von Alkoholen und Äthern.

(30) Priorität: 28.04.81 DE 3116779

(43) Veröffentlichungstag der Anmeldung:
03.11.82 Patentblatt 82/44

(45) Bekanntmachung des Hinweises auf die Patenterteilung:
02.01.85 Patentblatt 85/1

(84) Benannte Vertragsstaaten:
AT BE CH DE FR GB IT LI LU NL SE

(56) Entgegenhaltungen:
CA - A - 958 213
FR - A - 2 367 721
GB - A - 2 050 379
US - A - 3 849 082
US - A - 4 046 520

L'INDUSTRIE DU PETROLE, Band 48, Nr. 524, 1980,
Seiten 57-62, Paris, FR. G. JACQUES: "New
petrochemical processes"

Die Akte enthält technische Angaben, die nach dem
Eingang der Anmeldung eingereicht wurden und die
nicht in dieser Patentschrift enthalten sind.

(73) Patentinhaber: **VEBA OEL AG,
Alexander-von-Humboldt-Strasse,
D-4650 Gelsenkirchen-Hassel (DE)**

(72) Erfinder: **Gottlieb, Klaus, Dr., Alte Strasse 59,
D-5804 Herdecke-Ende (DE)**
Erfinder: **Bruderreck, Hartmut, Dr.,
Bärenkampstrasse 55, D-4660 Gelsenkirchen-Buer (DE)**
Erfinder: **Wehmeier, Friedel-Heinrich, Dr.,
Klaus-Groth-Strasse 1, D-4250 Bottrop-Kirchhellen (DE)**

(74) Vertreter: **Krug, Joachim, Dr.,
Alexander-von-Humboldt-Strasse,
D-4650 Gelsenkirchen-Hassel (DE)**

## Beschreibung

Die Erfindung betrifft ein Verfahren zur Herstellung von Alkoholen und Äthern aus unter Normalbedingungen gasförmigen Kohlenwasserstoffen, die z. B. bei der Rohölförderung oder -verarbeitung anfallen. Das Sammeln, Reinigen, Transportieren und Verteilen dieser Paraffinkohlenwasserstoffe ist sehr aufwendig und mit hohen Kosten verbunden. Die Verwendung des $C_3$- und $C_4$-Anteiles dieser Paraffinkohlenwasserstoffe als Kraftstoffe in Verbrennungsmaschinen ist, obwohl technisch möglich, deswegen energetisch ungünstig. Die insgesamt für das Sammeln, Reinigen, Transportieren und Verteilen aufzuwendende Energie ist im Verhältnis zur in herkömmlichen Verbrennungsmotoren erzielten Energieausnutzung zu groß, so daß langfristig der Einsatz dieser Paraffinkohlenwasserstoffe als Autogas ohne steuerliche Begünstigung unwirtschaftlich ist.

Der vorliegenden Erfindung liegt die Aufgabe zugrunde, diese Paraffinkohlenwasserstoffe, die heute sogar noch in größeren Mengen abgefackelt werden, mit höherer Energierentabilität in wertvolle Kraftstoffkomponenten umzuwandeln, deren Erzeugung, Transport und Verteilung einen geringeren Energieeinsatz verlangt als Sammeln, Reinigen, Transport und Verteilung der gasförmigen Kohlenwasserstoffe und deren Einsatz in herkömmlichen Verbrennungsmotoren ohne Zusatzausrüstung möglich ist und dort zur Verringerung des spezifischen und absoluten Kraftstoffverbrauchs sowie zur Umweltentlastung beiträgt.

Aus den DE-OS 2 620 011 und 2 921 576 ist es bekannt, Butan zu Methyl-tert.-butylether zu verarbeiten. Hierbei wird n-Butan partiell oder vollständig zu Isobutan (2-Methyl-propan) isomerisiert und das n-Butan-Isobutan-Gemisch dehydriert, wobei neben Isobuten auch n-Butene, Butadien, Propen und Propan gebildet werden. Das Dehydrierungsreaktionsgemisch wird dann mit einem Überschuß an Methylalkohol veräthert, wobei sich das in der Dehydrierungsstufe gebildete Isobuten zu Methyl-tert.-butylether umsetzt. Der überschüssige Methylalkohol aus dem Verätherungsreaktionsgemisch wird entweder mit Wasser oder durch Azeotrop-Destillation entfernt. In der Isomerisierung und Dehydrierung werden von 25 bis zu 30 Gew.-% der eingesetzten Butanfraktion zu n-Butan, Butadien, Propan, Propen, Äthan, Äthen, Methan und Wasserstoff umgewandelt. Diese Isomerisier- und Dehydrierabgase können in den vorbeschriebenen Verfahren nur als Brennstoff für Heizzwecke Verwendung finden, werden somit also erheblich abgewertet.

Hauptzweck des in OS 2 921 576 beschriebenen Verfahrens ist die Erzeugung von reinem Methyl-tert.-butylether bzw. des in OS 2 620 011 vorbeschriebenen Verfahrens die Herstellung von reinem Methyl-tert.-butylether oder reinem Methyl-tert.-amylether bzw. einem Gemisch dieser beiden reinen Äther aus Methanol und tert. Olefinen.

Aus den US-PS 3 904 384 und US-PS 4 046 520 ist es auch bekannt, Butane zu Isopropyl-tert.-butylether zu verarbeiten, wobei n-Butan isomerisiert und das erzeugte Isobutan nichtkatalytisch gecrackt wird, wobei neben Isobuten auch Propen gebildet wird. Das Propen wird zu Isopropanol und dieses zusammen mit dem gebildeten Isobuten zu Isopropyl-tert.-butylether umgewandelt. Auch diesem Verfahren haftet der große Nachteil an, daß ein großer Teil der eingesetzten Butanfraktion infolge des bei 640—670°C betriebenen Crackprozesses zu Produkten umgewandelt werden, die nur als Brennstoff für Heizzwecke eingesetzt werden können, wodurch die Gesamtenergierentabilität wiederum erheblich beeinträchtigt wird. Auch soll nach diesem Verfahren wiederum nur reiner Isopropyl-tert.-butylether hergestellt werden.

Im Gegensatz hierzu ist das Ziel vorliegender Erfindung, nicht Reinkomponenten, sondern Gemische aus gegebenenfalls Alkohol enthaltenden Äthern zu erzeugen, die in bestimmten Zusammensetzungen synergistische Effekte bei der Verbrennung im Motor zeigen und die, wie Experimente und Berechnungen zur Energienutzung aller eingesetzten und erzeugten Stoffe gezeigt haben, hinsichtlich des Gesamtenergieverbrauchs optimiert sind.

Hierzu soll nach der vorliegenden Erfindung die Umwandlung auch des bei der Isomerisierung und Dehydrierung anfallenden n-Butens, Butadiens, Propens und Propans sowie auch des in den Einsatzkohlenwasserstoffen enthaltenen Propans und eines Teils des n-Butans zu $C_3$- und $C_4$-Alkoholen und mit Isobuten zu den auf $C_3$- und $C_4$-Alkoholen aufbauenden Äthern erfolgen, wobei auch ein Teil des Isobutens mit Methylalkohol, der aus Methan und Äthan hergestellt wird, in der bekannten Weise zu Methyl-tert.-butylether verarbeitet werden kann.

Hierfür werden gemäß vorliegender Erfindung aus dem Propan und Butan enthaltenden Gemisch leichter Kohlenwasserstoffe eine Propan- und eine Butanfraktion abgetrennt. Propan wird katalytisch dehydriert und das entstandene Propen mit Wasser zu Isopropylalkohol (Propan-2-ol) umgesetzt. In der Butanfraktion wird ein Teil des n-Butans isomerisiert, katalytisch dehydriert und das im Dehydrierungsreaktionsgemisch enthaltene Isobuten zumindest teilweise mit einem Gemisch des aus der Propanfraktion gewonnenen Isopropylalkohols und des aus einem Teil der Butanfraktion gewonnenen sec.-Butylalkohols zu einem Gemisch aus sec.-Butyl-tert.-butylether und Isopropyl-tert.-butylether umgesetzt. Die nicht umgesetzten Kohlenwasserstoffe aus der Propen-Hydratisierung sowie nicht umgesetztes Isobutan aus der Verätherung werden in die Dehydrierstufe zurückgeführt.

In dem Dehydrierungsreaktionsgemisch wird Butadien selektiv unter gleichzeitiger Umwandlung von Buten-1 zu Buten-2 hydriert und nach Umsetzung des im Dehydrierungsreaktionsgemisch enthaltenen Isobutens mit einem Gemisch aus rückgeführtem sec.-Butylalkohol und Isopropylalkohol zu Isopropyl-tert.-butylether-sec.-Butyl-tert.-butylether-Gemisch Buten-2 des vorwiegend Buten-2 und Butan enthaltenden Kohlenwasserstoffgemisches nach der Verätherung mit Wasser zu sec.-Butylalkohol umgesetzt. Die nicht umgesetzten Kohlenwasserstoffe aus der Buten-2-Hydratisierung werden in die Dehydrierstufe zurückgeführt.

Die Erfindung gestattet auch die Gewinnung von tert.-Butylalkohol (2-Methyl-Propan-2-ol), im Gemisch mit Isopropylalkohol, Isopropyl-tert.-butylether, sec.-Butylalkohol und sec.-Butyl-tert.-butylether aus den Wasser enthaltenden Alkoholen und Isobuten.

Schließlich erlaubt die vorliegende Erfindung auch, neben den erwähnten Alkoholen und Äthern Methylalkohol und Methyl-tert.-butylether zu gewinnen. Hierfür wird neben den Propan- und Butanfraktionen eine Methan und Äthan enthaltende Fraktion von dem als Rohstoff benutzten Kohlenwasserstoffgemisch abgetrennt und mit dem bei der Dehydrierung von Butan und Propan anfallenden Strom an Methan, Äthan und Äthen zusammengeführt. Aus diesem Gemisch wird durch Reformieren mit Wasserdampf zum Beispiel unter einem Druck von 40–100 bar und Temperaturen von 210–300°C in bekannter Weise katalytisch Methylalkohol hergestellt und dieser mit einem Teil des im Dehydrierungsreaktionsgemisch enthaltenen Isobutens zu Methyl-tert.-butylether umgesetzt.

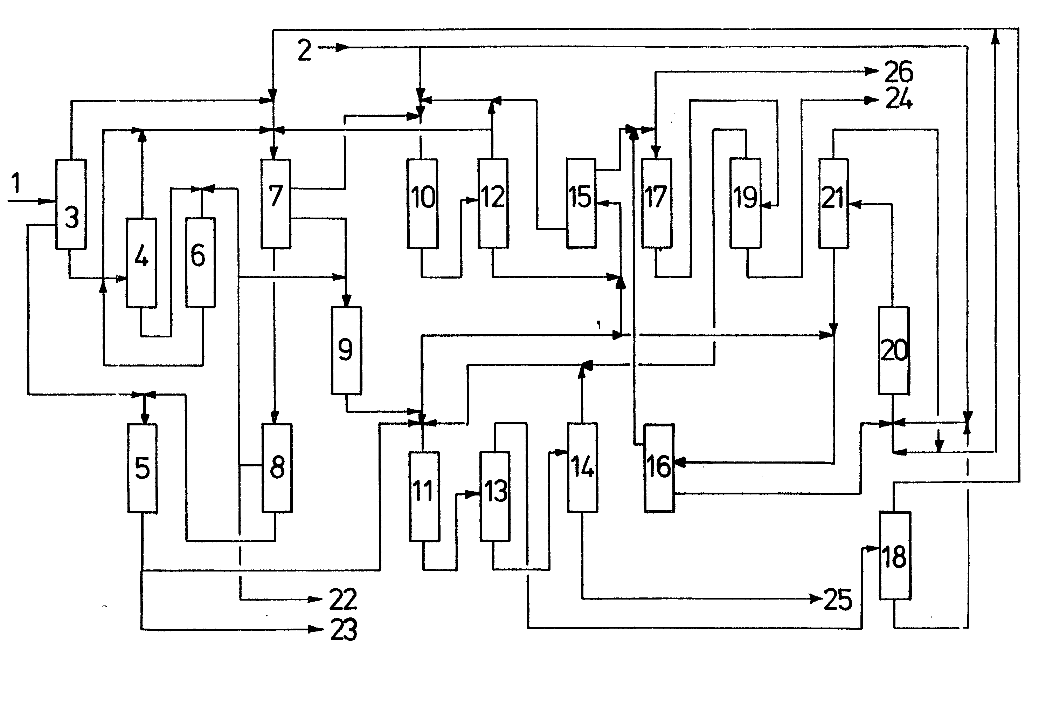

Weitere Ausbildungen der Erfindung ergeben sich aus der nachfolgenden Verfahrensbeschreibung, in der das Verfahren unter Bezugnahme auf die in der zugehörigen Zeichnung enthaltene Prinzipskizze näher erläutert wird. Die Zeichnung stellt eine bevorzugte Ausführungsform des erfindungsgemäßen Verfahrens unter Weglassung der für das Verständnis nicht erforderlichen Teile wie Pumpen, Wärmetauscher und einiger Destillationskolonnen dar.

Das Kohlenwasserstoffgemisch 1 wird durch Destillation 3 in eine Butan, eine Propan und eine Methan und Äthan enthaltende Fraktion aufgetrennt. Die Isomerisierung 6 — n-Butan zu Isobutan — erfolgt in an sich bekannter Weise an einem Platin-haltigen Festbettkatalysator in Gegenwart von Wasserstoff bei Temperaturen von 150–210°C und Drücken von 15–30 bar. Die Reaktionsbedingungen hinsichtlich Druck und Temperatur werden insgesamt so eingestellt, daß das Isomerisierungsgleichgwicht möglichst erreicht wird.

Von dem den Isomerisierungsreaktor 6 verlassenden, zu mehr als 50% aus Isobutan bestehenden Reaktionsgemisch werden Wasserstoff und bei der Isomerisierung entstandenes Methan, Äthan und Propan abgetrennt, das $C_4$-Isomerisat wird mit der eingesetzten Butanfraktion vereinigt. Ein aliquoter Teil des Isobutan-n-Butan-Gemisches wird in einer Destillationskolonne 4 mit 30–100 Böden unter einem Druck von 7–14 bar und bei Temperaturen von 40–80°C rektifiziert, so daß das über Kopf entnommene Isobutan-n-Butan-Gemisch einen Gehalt von 80–98% Isobutan aufweist. Das dem Sumpf der Destillationskolonne 4 entnommene n-Butan wird in die Isomerisierung 6 zurückgeführt. Das Kopfprodukt der Destillationskolonne 4 wird mit dem restlichen Teil der eingesetzten Butanfraktion vereinigt und zusammen mit dem rückgeführten Isobutan aus den Verätherungen 11 und 17 und den rückgeführten Kohlenwasserstoffen aus der Hydratisierung 20 der Dehydrierung 7 zugeführt.

Durch die Isomerisierung und Destillation wird n-Butan in einer solchen Menge in Isobutan überführt, wie als vorbestimmte stöchiometrische Menge für die Verätherung der insgesamt erzeugten Alkoholmenge notwendig ist. Der Isobutangehalt des Butanstromes nach Isomerisierung und Destillation beträgt 40–98 Gew.-%, vorzugsweise 55–90 Gew.-%.

Der Propanstrom kann mit dem aus der Hydratisierung 10 rückgeführten Propan vereinigt und einer getrennten Dehydrierstufe zugeführt werden. In der bevorzugten Ausführungsform des erfindungsgemäßen Verfahrens wird die Propanfraktion mit den aus der Hydratisierung 10 rückgeführten Kohlenwasserstoffen vereinigt und zusammen mit der erfindungsgemäß hergestellten Butan-Beschickung der gemeinsamen Dehydrierung 7 zugeführt.

Die Dehydrierung der $C_4$- und $C_3$-Kohlenwasserstoffe erfolgt in an sich bekannter Weise katalytisch, wahlweise in einem Festbett- oder einem Fließbettreaktor. Die Dehydriertemperatur liegt zwischen 530 und 700°C, der Druck zwischen 0,2 und 5 bar, vorzugsweise zwischen 0,3 und 1,5 bar. Der Dehydrierungskatalysator besteht im allgemeinen aus aktivem Aluminiumoxid und Zusätzen aus Chromoxid oder Platin, die durch Tränken auf $Al_2O_3$ aufgebracht werden.

Der während der Reaktionsphase entstehende Koks wird in einer Regenerierphase mit Luft abgebrannt, die dabei freiwerdende Wärme wird als Prozeßwärme zurückgewonnen. Die Dehydrierungsreaktionsgemische werden durch Abkühlen und Komprimieren in einen gasförmigen, vorwiegend Methan, Äthan, Äthylen und den Wasserstoff enthaltenden Strom und flüssige, vorwiegend Propan und Propen bzw. Butane, Butadien und Butene enthaltende Ströme aufgetrennt.

Aus dem gasförmigen Strom wird der Wasserstoff in der Reinigungsanlage 8 in an sich bekannter Weise weitgehend abgetrennt. Methan, Äthan und Restwasserstoff werden zusammen mit der Methan- und Äthan-haltigen Fraktion der Methylalkohol-Erzeugungsanlage 5 zugeführt. Wenn keine adäquate Verwendungsmöglichkeit für den Gesamtwasserstoff besteht, wird der Methan- und Äthanhaltigen Fraktion des Reaktionsgemisches nach Dehydrierung nur so viel Wasserstoff entnommen, wie für die Isomerisierungs- und Hydrierungsreaktionen notwendig ist. Der Restwasserstoff kann mit dem Dehydrierabgas bei 22 zur Prozeßenergieerzeugung entnommen werden.

Wird nach der bevorzugten Ausführungsform des erfindungsgemäßen Verfahrens mit gemeinsamer Dehydrierung 7 von Propan und Butan gearbeitet, so wird das die $C_3$- und $C_4$-Kohlenwasserstoffe enthaltende Dehydrierungsreaktionsgemisch in einer Rektifizierungskolonne in einen Propan- und Propen-haltigen Strom sowie einen vorwiegend die Butane, Butene und Butadien enthaltenden Strom aufgetrennt.

Der alle $C_4$-Kohlenwasserstoffe enthaltende Strom wird einer Selektivhydrierung 9 und Hydroisomerisierung zugeführt, wodurch Butadien selektiv zu Buten hydriert und gleichzeitig alles Buten-1 in Buten-2 überführt wird. Die Selektivhydrierung und Hydroisomerisierung erfolgt in an sich bekannter Weise, katalytisch in Gegenwart von Wasserstoff in einem Festbettreaktor. Die Temperatur beträgt 20–80° C, vorzugsweise 30–60° C, der Druck 1–20 bar, vorzugsweise 1,5–10 bar. Der verwendete Katalysator besteht im allgemeinen aus einem Träger, z. B. Aluminiumoxid oder Siliziumoxid und Zusätzen aus Platin, Palladium oder Nickel.

Die Wasserstoffkonzentration und die Beschickungsgeschwindigkeit werden so gewählt, daß Butadien nahezu vollständig (Restgehalt an Butadien im Reaktionsgemisch kleiner 0,5 Gew.-%) und Buten-1 in maximaler Ausbeute nahe dem thermodynamischen Gleichgewichtswert so in Buten-2 überführt werden, daß Butene in möglichst geringer Menge (weniger als 10 Gew.-%) zu n-Butan hydriert werden.

Zweck dieser Stufe ist es, nach der Verätherung Isobutan destillativ so abtrennen zu können, daß Butene und n-Butan im Sumpf der Kolonne verbleiben. Die Siedelücke zwischen Isobutan einerseits, n-Butan und Buten andererseits ist so groß, daß eine einfache destillative Abtrennung des Isobutans möglich ist, wenn zuvor durch Hydroisomerisierung Buten-1 in Buten-2 überführt und Isobuten durch Verätherung abgetrennt wurde. In einer besonderen Ausführung des erfindungsgemäßen Verfahrens wird die Selektivhydrierung und Hydroisomerisierung nach der Verätherung angeordnet; dies ist dann vorzuziehen, wenn der Butadiengehalt der $C_4$-Fraktion kleiner ist als 2 Gew.-%, da in diesem Falle die in geringen Mengen aus Butadien gebildeten polymeren Stoffe bei der Verätherung sich nicht störend bemerkbar machen.

Die aus dem Dehydrierungsgemisch abgetrennte Propen-haltige $C_3$-Fraktion wird dem Hydratisierungsreaktor 10 zugeführt, in dem durch katalytische Synthese von Propen und Wasser 2 bei einem Druck von 30–100 bar, vorzugsweise bei 40–80 bar, und Temperaturen von 100–170° C, vorzugsweise 130–160° C, Isopropylalkohol hergestellt wird. Als Katalysatoren dienen saure Katalysatoren, bevorzugt stark saure Kationenaustauscher, die aus sulfonierten, mit Divinylbenzol vernetzten Polystyrolharzen bestehen.

Im Beschickungsstrom werden auf 1 Mol Propen 1–20 Mole Wasser, vorzugsweise 3–8 Mole, eingesetzt. Die Raumgeschwindigkeit in Liter Beschickung pro Liter Katalysator und Stunde beträgt 0,3–25, vorzugsweise 0,5–10. Unter diesen Reaktionsbedingungen werden 10–70% des eingesetzten Propens unter Bildung von Isopropylalkohol und Diisopropylether umgesetzt.

Von dem Reaktionsendgemisch werden durch Destillieren die $C_3$-Kohlenwasserstoffe über Kopf der Destillierkolonne 12 abgetrennt; ein Teilstrom wird in den Hydratisierungsreaktor 10 und ein mengenmäßig kleinerer Teil in die Dehydrierung zurückgeführt. Das Isopropylalkohol-Wassergemisch wird, gegebenenfalls nach destillativer Anreicherung von Isopropylalkohol, mit einem als Extraktionsmittel für Isopropylalkohol geeigneten, mit Wasser nicht mischbaren und von Isopropylalkohol leicht abtrennbaren organischen Lösungsmittel vermischt. Es ist ein besonderes Merkmal dieser Erfindung, daß hierfür die im Verfahren erzeugten olefinhaltigen $C_3$- oder insbesondere $C_4$-Ströme verwendet werden.

Nach Trennung des Extraktionsgemisches in eine organische Phase und eine wäßrige Phase enthält die organische Phase 40–95% der entstandenen Isopropylalkoholmenge und 80–98% der Di-isopropylethermenge. Von der organischen Phase werden durch Destillation die Kohlenwasserstoffe abgetrennt und in die Extraktion 15 rückgeführt. Der dem Sumpf entnommene Isopropylalkohol wird einschließlich des gebildeten Di-isopropylethers der Verätherung 17 zugeführt.

In der bevorzugten Ausführungsform wird zur Extraktion 15 die Isobuten enthaltende $C_4$-Fraktion eingesetzt. Dazu wird ein Gewichtsteil des aus dem Sumpf der Kolonne 12 abgezogenen Wasser-Isopropylalkohol-Gemisches mit 1–20 Gewichtsteilen der $C_4$-Fraktion vermischt und der Extraktionsstufe 15 zugeführt, wo das Gesamtgemisch in eine wäßrige und eine organische Phase aufgetrennt wird. Die organische Phase enthält 20–60 Gew.-% des der Extraktion zugeführten Isopropylalkohols und geringe Mengen Wasser; hieraus wird durch Destillieren ein Teil der Isobuten enthaltenden $C_4$-Fraktion abgetrennt, so daß der Rückstand neben wenig Wasser Isobuten und Isopropylalkohol in dem für die Verätherung 17 notwendigen stöchiometrtischen Verhältnis enthält. Soll neben Isopropyl-tert.-butylether-sec.-Butyl-tert.-butylether-Gemisch auch ein Isopropylalkohol-sec.-Butylalkohol-Gemisch erzeugt werden, so wird die organische Phase durch Destillieren vollständig in Isopropylalkohol und die Isobuten enthaltende $C_4$-Fraktion aufgetrennt und ein Isopropylalkohol-sec.-Butylalkohol-Gemisch aus dem Sumpf der Rektifizierkolonne bei 26 abgezogen. Die Verätherung zur Herstellung des Isopropyl-tert.-butylether-sec.-Butyl-tert.-butylether-Gemisches wird in diesem Fall mit getrennten Strömen für Isopropylalkohol-sec.-Butylalkohol-Gemisch und Isobuten enthaltender $C_4$-Fraktion beschickt. Die wäßrige Phase wird in die Hydratisierung 10 rückgeführt.

Um die Trennleistung der Extraktionsstufe zu erhöhen, kann aus dem am Sumpf der Kolonne 12 abgezogenen Isopropylalkohol-Wasser-Gemisch durch Destillieren zunächst ein an Isopropylalkohol angereichertes Isopropylalkohol-Wasser-Gemisch abgetrennt und wie vorgehend beschrieben mit der Isobuten enthaltenden $C_4$-Fraktion behandelt werden. Der Anreicherungsgrad kann bis zu 88 Gew.-% betragen. Zur extraktiven Abtrennung von Isopropylalkohol werden dann ein Gewichtsanteil des an Isopropylalkohol angereicherten wäßrigen Gemisches mit 1—5 Gewichtsteilen Isobuten enthaltender C₄-Fraktion gemischt und der Extraktionsstufe 15 zugeführt, wo eine 70—95 Gew.-% des in der Hydratisierung gebildeten Isopropylalkohols enthaltende organische Phase abgetrennt wird. Wasser wird wieder in die Hydratisierung zurückgeführt.

In einer besonderen Ausführungsform des erfindungsgemäßen Verfahrens kann ein nach der Hydratisierung 10 über Kopf der Anreicherungskolonne abgenommene Isopropylalkohol-Wassergemisch mit dem entsprechenden sec.-Butylalkohol-Wassergemisch vereinigt, direkt der Verätherung 17 zugeführt und ein tert.-Butylalkohol-haltiges Äther-Alkoholgemisch hergestellt und bei 24 abgezogen werden.

Isobuten und ein Isopropylalkohol-sec.-Butylalkohol-Gemisch werden katalytisch veräthert, wobei Alkohol zu 10—100%, vorzugsweise zu 50—90%, unter Bildung von Isopropyl-tert.-butylether und sec.-Butyl-tert.-butylether sowie geringer Mengen Di-isopropylether umgesetzt wird. In kleinen Mengen werden tert.-Butylalkohol und Trimethylpentene gebildet. Es wurde gefunden, daß n-Butene keine Umsetzung eingehen, und den Verätherungsreaktor 17 unverändert verlassen. Als saure Katalysatoren dienen sulfonierte Kationenaustauscherharze, bevorzugt wiederum stark saure Ionenaustauscher auf der Basis von sulfoniertem, mit Divinylbenzol vernetztem Styrol. Die Verätherung erfolgt in der Flüssigphase in einem ein- oder mehrstufigen Festbettreaktor 17 bei Temperaturen zwischen 20—150° C, vorzugsweise bei 30—60° C, und Drücken, die zur Verflüssigung des Isobutens ausreichen, nämlich von 4—40 bar, vorzugsweise 8—16 bar. Das Molverhältnis von Alkohol zu Isobuten soll im Bereich von 1 : 0,5 bis 1 : 10, vorzugsweise von 1 : 1 bis 1 : 3, die Raumgeschwindigkeit, ausgedrückt in Liter Beschickungsprodukt pro Liter Katalysator und Stunde in dem Bereich von 0,3—50, vorzugsweise 1—20, liegen. Der den Verätherungsreaktor 17 verlassende Strom besteht vorwiegend aus Isopropyl-tert.-butylether, sec.-Butyl-tert.-butylether, nicht umgesetzten Isobuten, Isopropylalkohol, sec.-Butylalkohol sowie Buten und Butan.

Zur Abtrennung des Isopropyl-tert.-butylether-sec.-Butyl-tert.-butylether-Gemischs wird das Verätherungsreaktionsgemisch einer Druckdestillationskolonne 19 zugeführt. Die nicht umgesetzten Isobuten-haltigen C₄-Kohlenwasserstoffe können zur Erzielung eines höheren Isobuten-Gesamtumsatzes in die Verätherung 17 zurückgeführt werden. Wird in der gewählten Ausführungsform auch Methyl-tert.-butylether durch Methylalkohol-Verätherung hergestellt, so werden die nicht umgesetzten, Isobuten enthaltenden C₄-Kohlenwasserstoffe zweckmäßigerweise der Methylalkohol-Verätherung 11 zugeführt.

Das Äther-Alkohol-Gemisch aus dem Sumpf der Kolonne 19 kann durch Waschen mit Wasser in eine Wasser-Alkoholphase und eine Ätherphase getrennt werden. Dazu wird einem Gewichtsteil Äther-Alkoholgemisch 1—20 Gewichtsteile Wasser, vorzugsweise 5—10 Gewichtsteile Wasser zugeführt, und das ganze bei 15—50° C, vorzugsweise bei 20—40° C intensiv gemischt. Die Trennung in Ätherphase und Wasser-Alkoholphase kann beispielsweise nach dem Mixer-Settler-Prinzip erfolgen. Das abgetrennte Ätherraffinat enthält 0,5—3 Gew.-% Trimethylpentene und jeweils weniger als 1 Gew.-% Isopropylalkohol, 1 Gew.-% Isobuten, 0,5 Gew.-% tert.-Butylalkohol und 0,2 Gew.-% Wasser.

Das für die Wasserwäsche notwendige Wasser setzt sich zu einem Teil aus dem Rückführwasser der Extraktion 15 und zum anderen Teil aus dem für die Hydratisierung notwendigen Frischwasser 2 zusasmmen. Die aus der Wasserwäsche abgezogene Alkohol enthaltende Wasserphase kann in die Extraktion 16 zurückgeführt und zusammen mit dem Reaktionsgemisch aus dem Hydratisierungsreaktor 22, wie später beschrieben, aufgearbeitet werden.

Soll nach der bevorzugten Ausführungsform ein Äther-Alkohol-Gemisch hergestellt werden, so wird das Isopropylalkohol-sec.-Butylalkohol-Gemisch mit einem solchen Überschuß an Isobuten veräthert, daß eine Abtrennung der nicht umgesetzten Alkohole unterbleiben kann. Am Sumpf der Druckkolonne 19 wird dann bei 24 ein Äther-Alkohol-Gemisch abgezogen.

Aus der DE-OS 2 535 471 sowie US-PS 4 046 520 und CA-PS 958 213 ist die Herstellung von Isopropyl-tert.-butylether an sich bekannt. In der OS 2 535 471 ist auch die Herstellung von sec.-Butyl-tert.-butylether beispielhaft beschrieben. Im Unterschied zu den dort beschriebenen Ausführungsformen, die alle auf einen während der Reaktion vorhandenen Überschuß an Isopropylalkohol bzw. sec.-Butylalkohol und damit notwendigerweise auf höhere Temperaturen abheben, wird in der vorliegend beschriebenen bevorzugten Ausführungsform mit einem Überschuß an Isobuten und niedrigeren Temperaturen gearbeitet, wodurch ein höherer Alkohol-Umsatz erreicht wird und damit auf eine Abtrennung und Rückführung des nicht umgesetzten Alkohols verzichtet werden kann. Auch für die vorgehend beschriebene Ausführungsform, in der nicht umgesetzter Alkohol durch Behandeln mit Wasser abgetrennt und zurückgeführt wird, ist es wirtschaftlicher, mit einem Isobutenüberschuß und höchstmöglichem Alkohol-Umsatz zu arbeiten.

Ein weiterer Vorteil der Arbeitsweise mit niedrigen Reaktionstemperaturen ist, daß eine Dehydratisierung des Isopropylalkohols und sec.-Butylalkohols in Wasser und Propen bzw. n-Buten-2 vermieden wird, was die Wirtschaftlichkeit der Verfahren bei höheren Reaktionstemperaturen nachteilig beeinflußt. Es wurde auch beobachet, daß der Isopropylalkohol- und sec.-Butylalkohol-Umsatz bei der Verätherung eines Gemischs derselben geringfügig höher sind als bei der Verätherung der reinen Alkohole, was auf eine für die Reaktion günstigere Verteilung an der Katalysatoroberfläche zurückzuführen ist.

In einer besonderen Ausführungsform des erfindungsemäßen Verfahrens wird die Isobuten-haltige $C_4$-Fraktion mit einem sec.-Butylalkohol-Isopropylalkohol-Wassergemisch in Gegenwart der bereits beschriebenen sauren Katalysatoren umgesetzt, wobei Alkohol zu 10—95%, vorzugsweise zu 50—90%, unter Bildung von Isopropyl-tert.-butylether und Wasser zu 80—100%, unter Bildung von tert.-Butylalkohol mit Isobuten umgesetzt werden. Das als Beschickung verwendete Alkohol-Wassergemisch kann 1—50 Gew.-% Wasser enthalten, insbesondere kann man ein bei einer Azeotrop-Destillation, z. B. das bei der beschriebenen Isopropylalkohol- und sec.-Butylalkohol-Anreicherung anfallende Alkohol-Wasser-Gemisch einsetzen.

Es wurde überraschenderweise gefunden, daß auch in Gegenwart von Wasser die n-Butene keine Umsetzung eingehen. Überraschenderweise wurde weiterhin festgestellt, daß tert.-Butylalkohol kein Reaktionsprodukt durch Parallelreaktion mit Isobuten bildet. Als Katalysatoren können dieselben sulfonierten stark sauren Ionenaustauscher dienen, die auch in der vorher beschriebenen Ausführungsform verwendet werden. Die Verätherung erfolgt in einem mehrstufigen Festbettreaktor bei Temperaturen zwischen 20 und 150°C, vorzugsweise bei 30—80°C und Drücken, die zur Verflüssigung von Isobuten ausreichen, also von 4—40 bar, vorzugsweise 8—16 bar. Das Molverhältnis von Alkohol zu Isobuten liegt im Bereich von 1 : 0,1 bis 1 : 10, vorzugsweise von 1 : 1 bis 1 : 5, das Molverhältnis von Wasser zu Isobuten im Bereich von 1 : 1 bis 1 : 20, vorzugsweise von 1 : 1,5 bis 1 : 10, die Raumgeschwindigkeit in Liter Beschickungsprodukt pro Liter Katalysator und Stunde im Bereich von 0,3—50, vorzugsweise 1—20. Das Äther-Alkohol-Gemisch wird durch Destillation unter Druck von den nicht umgesetzten Kohlenwasserstoffen abgetrennt, welcher ihrerseits rückgeführt werden, wie es bei der vorgehenden Ausführungsform der Verätherung des Isopropylalkohol-sec.-Butylalkohol-Gemischs ohne Wasserzusatz beschrieben wurde.

Die an sich bekannte Herstellung von tert.-Butylalkohol aus Isobuten und Wasser an sauren Ionenaustauschern zusammen mit der Herstellung von Äthern aus Isobuten und Alkohol im gleichen Reaktionsschritt ist bisher noch nicht beschrieben. Der besondere Wert dieser Verfahrensvariante beruht darauf, daß bei sehr niedrigen Temperatuen und einem auf Wasser bezogenen Isobutenüberschuß ein nahezu 100prozentiger Umsatz von Wasser zu tert.-Butylalkohol, ohne Bildung nennenswerter Mengen an Isobutenoligomeren erzielt wird. In den vorbeschriebenen Verfahren von tert.-Butylalkohol muß dagegen mit einem auf Isobuten bezogenen Überschuß an Wasser gearbeitet werden, um den Zwangsfall an Isobutenoligomeren niedrig zu halten. Auch muß, um eine ausreichende Ausbeute an tert.-Butylalkohol zu erreichen, bei hohen Temperaturen gearbeitet und der anfallende tert.-Butylalkohol vom Wasser getrennt werden, was sehr hohe Trennkosten mit sich bringt. Das nach der besonderen Ausführungsform des erfindungsgemäßen Verfahren im Sumpf der Kolonne 19 anfallende tert.-Butylalkohol enthaltende Äther-Alkohol-Gemisch kann durch destillative Maßnahmen in ein tert.-Butylalkohol-reiches und ein tert.-Butylalkohol-freies Äther-Alkohol-Gemisch getrennt werden.

Ein weiterer Teilstrom der Isobuten enthaltenden $C_4$-Fraktion wird gegebenenfalls mit den $C_4$-Kohlenwasserstoffabströmen aus der Verätherung 17 von Isopropylalkohol-sec.-Butylalkohol-Gemisch vereinigt und das darin enthaltende Isobuten mit Methylalkohol katalytisch veräthert, wobei das Isobuten nahezu quantitativ zu Methyl-tert.-butylether umgesetzt wird. Als Katalysatoren dienen im allgemeinen die schon beschriebenen sulfonierten stark sauren Ionenaustauscherharze. Die Verätherung erfolgt ebenfalls in der Flüssigphase in einem Festbettreaktor 11. Die Temperaturen liegen in dem Bereich von 30 bis 100°C, vorzugsweise 60 bis 90°C, die Drücke bei 4 bis 24 bar, vorzugsweise 10 bis 20 bar. Bei der Verätherung wird Methylalkohol im Überschuß verwendet, um Isobuten möglichst weitgehend umzusetzen. Das Molverhältnis von Methylalkohol zu Isobuten liegt im allgemeinen im Bereich von 1 : 1 bis 2 : 1, vorzugsweise im Bereich von 1,1 : 1 bis 1,5 : 1. Der den Verätherungsreaktor 11 verlassende Strom besteht im wesentlichen aus Methyl-tert.-butylether, Isobutan, n-Butan, Butenen und Methylalkohol.

Zur Abtrennung des Methyl-tert.-butylethers wird das Gemisch der Druckdestillationskolonne 13 zugeführt, in der die nicht umgesetzten $C_4$-Kohlenwasserstoffe n-Butan, Isobutan und Butene über Kopf abgenommen werden und einer zweiten Destillationskolonne 18 zugeführt werden, wo Isobutan von den restlichen $C_4$-Kohlenwasserstoffen — n-Butan und Buten — abgetrennt wird. Isobutan wird in die Dehydrierung 7 zurückgeführt, die im Sumpf abgenommene n-Butan- und Butenfraktion wird in die Buten-Hydratisierung 20 geführt. Das aus dem Sumpf der ersten Rektifizierkolonne 13 abgezogene Methylalkohol-Äther-Gemisch wird in einer weiteren Druckdestillationskolonne 14 destilliert, wobei ein Äther-Methylalkohol-Azeotrop über Kopf geht und in die Verätherung 11 zurückgeführt wird, während aus dem Sumpf Methyl-tert.-butylether bei 25 abgezogen wird. Auch hier ist es nicht notwendig, bis zum reinen Methyl-tert.-butylether zu destilieren, wenn ein Alkohol-Äther-Gemisch hergestellt werden soll.

Nach der bevorzugten Ausführungsform des erfindungsgemäßen Verfahrens veräthert man das Isobuten-haltige $C_4$-Kohlenwasserstoffgemisch mit Methylalkohol, der durch Reformierung leichter Kohlenwasserstoffe mit Wasserdampf und durch katalytische Synthese unter einem Druck von 40 bis 100 bar, bei Temperaturen von 210—300°C hergestellt wurde. Als Einsatz für die Methylalkohol-Synthese wird die Methan- und Äthan-haltige Fraktion der bei der Rohölförderung und -verarbeitung anfallenden leichten Kohlenwasserstoffe eingesetzt. Soll im Rahmen des Verfahrens auch Methylalkohol gewonnen werden, so wird dieser bei 23 abgezogen.

Die nach der Methylalkohol-Verätherung abgetrennte, nur n-Buten und n-Butan enthaltende $C_4$-Fraktion wird der Buten-Hydratisierung 20 zugeführt, wo durch katalytische Synthese aus Buten und Wasser bei einem Druck von 20 bis 80 bar und 100—170°C, vorzugsweise bei 30 bis 60 bar und 120—160°C sec.-Butylalkohol hergestellt wird. Als Katalysatoren werden dieselben stark sauren Ionenaustauscher verwendet, die auch bei der Hydratisierung von Propen eingesetzt werden. Im Beschickungsstrom werden auf 1 Mol Buten 2—10 Mole, vorzugsweise 3—6 Mole Wasser eingesetzt. Die Raumgeschwindigkeit in Liter Beschickung pro Liter Katalysator und Stunde beträgt 0,2—15, vorzugsweise 0,5—5. Unter diesen Reaktionsbedingungen werden 5—35% des eingesetzten n-Butens unter Bildung von sec.-Butylalkohol und Spuren an Di-sec.-butylether umgesetzt. Von dem Reaktionsendgemisch werden durch Destillieren die $C_4$-Kohlenwasserstoffe über Kopf der Kolonne 21 abgetrennt und ein Teilstrom in den Hydratisierungsreaktor 20, ein kleinerer Teil in die Dehydrierung 7 zurückgeführt. Das sec.-Butylalkohol-Wasser-Gemisch, das gegebenenfalls mit dem in der Wasserwäsche der Verätherung 17 anfallenden sec.-Butylalkohol-Isopropylalkohol-Wasser-Gemisch vereinigt wird, wird gegebenenfalls nach destillativer Anreicherung von gegebenenfalls Isopropylalkohol enthaltendem sec.-Butylalkohol mit einem als Extraktionsmittel für sec.-Butylalkohol und Isopropylalkohol geeigneten, mit Wasser nicht mischbaren und von gegebenenfalls Isopropylalkohol enthaltendem sec.-Butylalkohol leicht abtrennbaren organischen Lösungsmittel vermischt. Gemäß einer besonderen Ausbildung dieser Erfindung wird dafür einer der im Verfahren erzeugten n-Buten- oder Isobuten enthaltenden $C_4$-Ströme verwendet. Nach Trennen des Extraktionsgemisches in eine organische Phase und eine wäßrige Phase enthält die organische Phase 50—98% der entstandenen sec.-Butylalkoholmenge und 90—98% der Di-sec.-butylethermenge und gegebenenfalls Isopropylalkohol. Von der organischen Phase werden nach Destillation die $C_4$-Kohlenwasserstoffe abgetrennt und in die Extraktionsstufe 16 rückgeführt. Der aus dem Sumpf der Destillation entnommene gegebenenfalls Isopropylalkohol enthaltende sec.-Butylalkohol wird einschließlich des gebildeten Di-sec.-butylethers der Verätherung 17 zugeführt.

In der bevorzugten Ausführungsform wird zur Extraktion 20 die Isobuten enthaltende $C_4$-Fraktion eingesetzt. Dazu werden 1 Gewichtsteil des aus dem Sumpf der Kolonne 21 abgezogenen, gegebenenfalls Isopropylalkohol enthaltenden Wasser-sec.-Butylalkohol-Gemisches mit 2—10 Gewichtsteilen der $C_4$-Fraktion vermischt und der Extraktionsstufe 16 zugeführt, wo das Gesamtgemisch in eine wäßrige und eine organische Phase aufgetrennt wird. Die organische Phase enthält 50—80 Gew.-% des der Extraktion zugeführten sec.-Butylalkohols geringe Mengen Wasser und gegebenenfalls Isopropylalkohol. Durch Destillieren wird ein geringe Mengen Wasser enthaltendes Gemisch aus sec.-Butylalkohol, Isobuten und gegebenenfalls Isopropylalkohol enthaltender $C_4$-Fraktion abgetrennt, das Isobuten, sec.-Butylalkohol und gegebenenfalls Isopropylalkohol in der für die Verätherung 17 notwendigen stöchiometrischen Menge enthält. Soll neben Isopropyl-tert.-butylether-sec.-Butyl-tert.-butylether-Gemisch auch ein sec.-Butylalkohol-Isopropylalkohol-Gemisch erzeut werden, so wird die organische Phase durch Destillieren vollständig in Alkohole und die Isobuten enthaltende $C_4$-Fraktion aufgetrennt und Alkohol aus dem Sumpf der Rektifizierkolonne bei 26 abgezogen. Die Verätherung 17 wird in diesem Fall mit getrennten Strömen für sec.-Butylalkohol-Isopropylalkohol-Gemisch und Isobuten enthaltender $C_4$-Fraktion beschickt. Die in der Extraktion abgetrennte wäßrige Phase wird in die Hydratisierung 20 zurückgeführt.

Um die Trennleistung der Extraktionsstufe zu erhöhen, kann aus dem am Sumpf der Kolonne 21 abgezogenen gegebenenfalls Isopropylalkohol enthaltendem sec.-Butylalkohol-Wasser-Gemisch durch Destillieren zunächst ein an sec.-Butylalkohol angereichertes gegebenenfalls Isopropylalkohol enthaltendes sec.-Butylalkohol-Wasser-Gemisch abgetrennt und, wie vorgehend beschrieben, mit der Isobuten-enthaltenden $C_4$-Fraktion behandelt werden. Der Anreicherungsgrad kann bis zu 80 Gew.-% betragen. Zur extraktiven Abtrennung von gegebenenfalls Isopropylalkohol enthaltendem sec.-Butylalkohol werden dann 1 Gewichtsteil des an sec.-Butylalkohol angereicherten wäßrigen Gemisches mit 0,5—5 Gewichtsteilen Isobuten enthaltender $C_4$-Fraktion gemischt und der Extraktionsstufe 20 zugeführt, wo eine 80—98 Gew.-% des in der Hydratisierung gebildeten sec.-Butylalkohols enthaltende organische Phase abgetrennt wird. Wasser wird wieder in die Hydratisierung zugeführt. An Stelle der Isobuten enthaltenden $C_4$-Fraktion kann als Extraktionsmittel auch die von Isobuten befreite, Buten-2 und n-Butan enthaltende $C_4$-Fraktion aus dem Einsatz zur Buten-Hydratisierung 20 verwendet werden.

## Beispiel 1

Ein Isobuten enthaltendes Kohlenwasserstoffgemisch, dessen Zusammensetzung in Tabelle 1, Spalte 1, angegeben ist, wurde mit einem Gemisch aus Isopropylalkohol und sec.-Butylalkohol bei einer Temperatur von 40°C und einem Druck über dem Dampfdruck von Isobuten, so daß dieses als Flüssigkeit vorliegt, nämlich 16 bar, umgesetzt, wobei eine solche Menge an Isobuten enthaltender Kohlenwasserstofffraktion eingesetzt wurde, daß das molare Mengenverhältnis Isobuten zu Isopropylalkohol zu sec.-Butylalkohol = 1,5 : 0,5 : 0,5 betrug. Der mit Katalysator (Handelsprodukt Lewatit SPC 118) gefüllte Reaktor wurde mit 5 Gewichtsteilen des die Kohlenwasserstofffraktion, Isopropylal-

**0 063 815**

kohol und sec.-Butylalkohol enthaltenden Gemisches pro Stunde und pro Gewichtsteil getrockneten Katalysators beschickt. Zur Einstellung der genannten Temperatur wurde ein geeigneter Vorheizer verwendet. Die bei der Reaktion freiwerdende Wärme wurde über einen Kühler abgeführt. Das Reaktionsendgemisch wurde durch Destillation von den nicht umgesetzten Kohlenwasserstoffen weitgehend befreit und hatte die in Tabelle 1, Spalte 2, angegebene Zusammensetzung. Die Zusammensetzung der nicht umgesetzten Kohlenwasserstoffe ist in Spalte 3 der Tabelle 1 ausgewiesen.

Das stabilisierte Äther-Alkoholgemisch genannter Zusammensetzung wurde durch Waschen in einer zweistufigen Mixer-Settler-Anordnung mit jeweils der dreifachen Menge an Wasser von Isopropylalkohol und sec.-Butylalkohol weitgehend befreit. Die nach dieser Wasserwäsche anfallende Ätherphase hatte die in der Tabelle 1, Spalte 4, angegebene Zusammensetzung.

Tabelle 1

| Komponente | Gew.-% 1 | Gew.-% 2 | Gew.-% 3 | Gew.-% 4 |
|---|---|---|---|---|
| Propan | 0,4 | — | 0,4 | — |
| Propen | 0,1 | — | 0,1 | — |
| Isobutan | 34,6 | 0,3 | 45,0 | 0,3 |
| n-Butan | 15,8 | 0,4 | 19,1 | 0,4 |
| Buten-1 | 14,4 | 0,3 | 17,2 | 0,2 |
| Isobuten | 34,4 | 0,1 | 17,5 | 0,1 |
| Buten-2 | 0,1 | — | 0,1 | — |
| Isopentan | 0,3 | 0,1 | 0,3 | 0,1 |
| Isopropylalkohol | — | 2,9 | — | 0,2 |
| sec.-Butylalkohol | — | 6,2 | — | 1,9 |
| tert.-Butylalkohol | — | 1,0 | — | 1,1 |
| Isobutenoligomere | — | 0,9 | — | 1,0 |
| Isopropyl-tert.-butylether | — | 38,8 | — | 41,8 |
| sec.-Butyl-tert.-butylether | — | 48,7 | — | 52,9 |

Beispiel 2

Ein 1,25 Mol Wasser, 1 Mol Isopropylalkohol und 1 Mol sec.-Butylalkohol enthaltendes Gemisch wurde mit einem Isobuten enthaltendem $C_4$-Kohlenwasserstoffgemisch der in Tabelle 2, Spalte 1, angegebenen Zusammensetzung im molaren Mengenverhältnis Wasser zu Isopropylalkohol zu sec.-Butylalkohol zu Isobuten = 1,25 : 1 : 1 : 4,75 in einem aus zwei in Serie geschalteten Reaktoren bestehendem Reaktorsystem umgesetzt. Der erste Reaktor war ein schlanker Rohrreaktor mit einem Verhältnis von Innendurchmesser zu Länge von 1 : 30, der zweite Reaktor hatte ein Verhältnis von Innendurchmesser zu Länge von 1 : 10. Als Katalysator wurde ein stark saures Ionenaustauscherharz (Handelsprodukt Lewatit SPC 118) verwendet. Die Temperatur am Eintritt des ersten Reaktors betrug 70° C, am Eintritt des zweiten Reaktors 40° C. Der Druck war über dem Dampfdruck von Isobuten, so daß das $C_4$-Kohlenwasserstoffgemisch als Flüssigkeit vorlag, nämlich 16 bar, gewählt.

Das mit Katalysator befüllte Reaktorsystem wurde pro Stunde und pro Gewichtsteil getrockneten Katalysators mit 10,1 Gewichtsteilen genannten Gemisches aus Wasser, Isopropanol, sec.-Butylalkohol und Isobuten enthaltenden $C_4$-Kohlenwasserstoffen beschickt. Das Verhältnis des Katalysatorvolumens in Reaktor 1 zum entsprechenden Volumen in Reaktor 2 betrug 1 : 3. Zur Einstellung der genannten Temperaturen wurde vor dem ersten Reaktor ein geeigneter Vorheizer, nach dem ersten bzw. vor dem zweiten Reaktor ein geeigneter Kühler verwendet. Die Reaktionswärme wurde ebenfalls

8

0 063 815

durch einen geeigneten Kühler abgeführt.

Das Reaktionsendgemisch wurde durch Destillation von den nicht umgesetzten Kohlenwasserstoffen weitgehend befreit und hatte die in Tabelle 2, Spalte 2, angegebene Zusammensetzung. Die Zusammensetzung der nicht umgesetzten Kohlenwasserstoffe hatte die in Spalte 3 der Tabelle angegebene Zusammensetzung.

Tabelle 2

| Komponente | Gew.-% 1 | Gew.-% 2 | Gew.-% 3 |
|---|---|---|---|
| Propan | 4,7 | — | 6,0 |
| Isobutan | 33,1 | 0,7 | 43,0 |
| n-Butan | 12,0 | 0,4 | 15,0 |
| Buten-2 | 0,4 | — | 0,6 |
| Buten-1 | 4,2 | — | 5,5 |
| Isobuten | 45,0 | 0,4 | 29,6 |
| Isopentan | 0,6 | 0,7 | 0,2 |
| Isopropylalkohol | — | 4,9 | — |
| sec.-Butylalkohol | — | 8,0 | — |
| tert.-Butylalkohol | — | 29,3 | — |
| Isopropyl-tert.-butylether | — | 27,1 | — |
| sec.-Butyl-tert.-butylether | — | 27,3 | — |
| Isobutenoligomere | — | 0,8 | — |
| Wasser | — | 0,2 | — |

**Patentansprüche**

1. Verfahren zur Herstellung eines gegebenenfalls Isopropylalkohol und/oder sec.-Butylalkohol und/oder tert.-Butylalkohol enthaltenden Gemisches von Isopropyl-tert.-butylether und sec.-Butyl-tert.-butylether aus einem Gemisch leichter Kohlenwasserstoffe, die Propan und Butan enthalten, dadurch gekennzeichnet, daß eine Propan- und eine Butanfraktion abgetrennt werden, n-Butan isomerisiert wird, Butan und Propan katalytisch dehydriert werden, Butadien selektiv unter gleichzeitiger Umwandlung von Buten-1 zu Buten-2 hydriert wird, Propen aus dem vorwiegend Propen und Propan enthaltendem Gemisch mit Wasser zu Isopropylalkohol umgesetzt, ein Teil des im Dehydrierungsreaktionsgemisch enthaltenden Isobutens mit einem gegebenenfalls Wasser enthaltendem Gemisch aus Isopropylalkohol und rückgeführtem sec.-Butylalkohol zu einem Isopropyl-tert.-butylether und sec.-Butyl-tert.-butylether enthaltendem Gemisch umgesetzt, das Buten-2 aus dem nicht umgesetzten vorwiegend Buten-2 und Butan enthaltenden Kohlenwasserstoffgemisch mit Wasser zu sec.-Butylalkohol umgesetzt und dieser in die Verätherung zurückgeführt wird, während nicht umgesetzte Kohlenwasserstoffe aus der Propen- und Buten-2-Hydratisierung sowie nicht umgesetztes Isobutan aus der Verätherung in die Dehydrierstufe zurückgeführt werden.

2. Verfahren nach Anspruch 1, dadurch gekennzeichnet, daß die Propanfraktion gemeinsam mit der Butanfraktion dehydriert und aus dem erhaltenen Dehydrierungsreaktionsgemisch wieder eine $C_3$-Fraktion abgetrennt wird.

3. Verfahren nach Anspruch 1 oder 2, dadurch gekennzeichnet, daß aus einem sec.-Butylalkohol und Isopropylalkohol enthaltendem Gemisch und flüssigem Isobuten in Gegenwart saurer Katalysatoren bei Temperaturen von 20—150, vorzugsweise 30—60°C ein Isopropyl-tert.-butylether und sec.-Butyl-tert.-butylether enthaltendes Gemisch hergestellt wird, wobei auf ein 1 Mol Alkohol 0,5—10, vorzugs-

9

weise 1—3 Mole Isobuten eingesetzt werden.

4. Verfahren nach einem der Ansprüche 1 bis 3, dadurch gekennzeichnet, daß ein Gemisch aus nicht umgesetztem Isopropylalkohol und nicht umgesetztem sec.-Butylalkohol durch Behandlung des Verätherungsreaktionsgemisches mit Wasser abgetrennt wird.

5. Verfahren nach Anspruch 1 oder 2, dadurch gekennzeichnet, daß ein sec.-Butylalkohol-Isopropylalkohol-Wasser-Gemisch in Gegenwart saurer Katalysatoren mit flüssigem Isobuten bei Temperaturen von 20—150, vorzugsweise 30—80°C zu einem tert.-Butylalkohol, sec.-Butyl-tert.-butylether und Isopropyl-tert.-butylether enthaltendem Gemisch umgesetzt wird, wobei eine solche Isobutenmenge eingesetzt wird, daß auf 1 Mol Wasser 1—20 Mole Isobuten und auf 1 Mol Alkohol 0,1—10 Mole Isobuten entfallen.

6. Verfahren nach einem der Ansprüche 1 bis 5, dadurch gekennzeichnet, daß durch Rektifizieren aus dem Reaktionsgemisch der Hydratisierung ein Isopropylalkohol-Wasser-Gemisch abgetrennt, aus diesem durch Behandeln mit einer Isobuten enthaltenden $C_4$-Fraktion Isopropylalkohol extrahiert, daraus ein Isopropylalkohol und Isobuten sowie gegebenenfalls Wasser enthaltendes Gemisch abdestilliert und der Verätherung zugeführt wird, während die von Isopropylalkohol befreite $C_4$-Fraktion in die Extraktion und die wäßrige Phase aus der Extraktion in die Hydratisierung zurückgeführt werden.

7. Verfahren nach einem der Ansprüche 1 bis 6, dadurch gekennzeichnet, daß das Isomerisat der Butanfraktion in eine n-Butan- und eine Isobutanfraktion getrennt, die n-Butanfraktion in die Isomerisierung zurückgeführt wird, die Isobutanfraktion einschließlich des nach der Verätherung rückgeführten Isobutans und einschließlich der aus den Hydratisierungsstufen zurückgeführten Kohlenwasserstoffe dehydriert wird.

8. Verfahren nach einem der Ansprüche 1 bis 7, dadurch gekennzeichnet, daß durch Rektifizieren aus dem Reaktionsgemisch der Hydratisierung ein Wasser-sec.-Butylalkohol-Gemisch abgetrennt, aus diesem durch Behandeln mit einer Isobuten enthaltenden $C_4$-Fraktion sec.-Butylalkohol extraktiv abgetrennt, daraus ein sec.-Butylalkohol und Isobuten sowie gegebenenfalls Wasser enthaltendes Gemisch abdestilliert und der Verätherung zugeführt wird, während die von sec.-Butylalkohol befreite $C_4$-Fraktion in die Extraktion und die wäßrige Phase aus der Extraktion in die Hydratisierung zurückgeführt werden.

9. Verfahren nach einem der Ansprüche 1 bis 7, dadurch gekennzeichnet, daß aus dem Reaktionsgemisch der Hydratisierung durch Behandeln mit einem vorwiegend aus n-Butan und Buten-2 bestehenden Kohlenwasserstoffgemisch sec.-Butylalkohol extrahiert, daraus gegebenenfalls Wasser enthaltender sec.-Butylalkohol abdestilliert und der Verätherung zugeführt wird, während das von sec.-Butylalkohol befreite Kohlenwasserstoffgemisch anteilig in die Hydratisierung und die Extraktion, die wäßrige Phase aus der Extraktion in die Hydratisierung zurückgeführt werden.

10. Verfahren zur Herstellung von gegebenenfalls Isopropylalkohol und/oder sec.-Butylalkohol und/ oder tert.-Butylalkohol und/oder Methylalkohol enthaltenden Gemischen von Isopropyl-tert.-butylether und sec.-Butyl-tert.-butylether und Methyl-tert.-butylether aus einem Gemisch leichter Kohlenwasserstoffe, das neben $C_3$- und $C_4$-Kohlenwasserstoffen auch $C_1$- und $C_2$-Kohlenwasserstoffe enthält nach Anspruch 1, dadurch gekennzeichnet, daß Methan und Äthan von dem als Rohstoff benutzten Kohlenwasserstoffgemisch abgetrennt und mit dem bei der Dehydrierung von Butan und Propan anfallenden Strom an Methan, Äthan und Äthen zusammengeführt werden, durch Reformieren dieses Gemisches in an sich bekannter Weise mit Wasserdampf unter einem Druck von 40—100 bar und Temperaturen von 210—300°C katalytisch Methylalkohol hergestellt, aus dem Isopropylalkohol, Isopropyl-tert.-butylether, sec.-Butylalkohol, sec.-Butyl-tert.-butylether und gegebenenfalls tert.-Butylalkohol enthaltendem Verätherungsgemisch die nicht umgesetzte Isobuten-haltige $C_4$-Kohlenwasserstofffraktion abgetrennt und mit einem Teil des im Dehydrierungsgemisch enthaltenen Isobutens vereinigt werden und aus Methylalkohol und Isobuten in an sich bekannter Weise Methyl-tert.-butylether hergestellt wird.

## Claims

1. Process for producing a mixture of isopropyl tert-butyl ether and sec-butyl tert-butyl ether, which mixture may contain isopropyl alcohol and/or sec-butyl alscohol and/or tert-butyl alcohol, from a mixture of light hydrocarbons containing propane and butane, characterized in that a propane and a butane fraction are separated, n-butane is isomerized, butane and propane are catalytically dehydrogenated, butadiene is selectively hydrogenated while butene-1 is simultaneously converted into butene-2, the propene in the mainly propene and propane containing mixture is converted with water into isopropyl alcohol, a portion of the isobutene contained in the dehydrogenation reaction mixture is converted with a mixture of isopropyl alcohol and recycled sec-butyl alcohol, which mixture may contain water, into a mixture of isopropyl tert-butyl ether and sec-butyl tert-butyl ether, the butene-2 from the unconverted hydrocarbon mixture containing mainly butene-2 and butane is converted with water into sec-butyl alcohol and this is recycled to the etherification, while unconverted hydrocarbons from the hydration of propene and butene-2 and unconverted isobutane from the etherification are recycled to the dehydrogenation stage.

2. Process according to Claim 1, characterized in that the propane fraction is dehydrogenated together with the butane fraction and a $C_3$-fraction is again separated from the resulting dehydrogenation reaction mixture.

3. Process according to Claim 1 or 2, characterized in that a mixture of isopropyl tert-butyl ether and sec-butyl tert-butyl ether is produced from a mixture containing isopropyl alcohol and sec-butyl alcohol and liquid isobutene in the presence of acid catalysts at temperatures of 20 to 150, preferably 30 to 60° C, whereby 0.5 to 10, preferably 1 to 3 moles of isobutene are used per one mole of alcohol.

4. Process according to one of the Claims 1 to 3, characterized in that a mixture of unconverted isopropyl alcohol and unconverted sec-butyl alcohol is separated by treating the etherification reaction mixture with water.

5. Process according to Claim 1 or 2, characterized in that a mixture of sec-butyl alcohol and isopropyl alcohol and water is reacted with liquid isobutene in the presence of acid catalysts at temperatures of 20 to 150, preferably 30 to 80° C to form a mixture containing tert-butyl alcohol, sec-butyl tert-butyl ether and isopropyl tert-butyl ether, wherein as much isobutene is used as is needed to provide 1 to 20 moles of isobutene per 1 mole of water and 0.1 to 10 moles of isobutene per 1 mole of alcohol.

6. Process according to one of the Claims 1 to 5, characterized in that a mixture of isopropyl alcohol and water is separated from the hydration reaction mixture by rectification, isopropyl alcohol is extracted from this mixture by treatment with a $C_4$-fraction containing isobutene, a mixture containing isopropyl alcohol and isobutene and possibly water is distilled off from this extract and fed to the etherification, while the $C_4$-fraction freed of isopropyl alcohol is recycled to the extraction and the aqueous phase from the extraction is recycled to the hydration.

7. Process according to one of the Claims 1 to 6, characterized in that the isomerizate of the butane fraction is divided into a n-butane fraction and an isobutane fraction, the n-butane fraction is recycled to the isomerization, the isobutane fraction, including the isobutane which is recycled from the etherification and the hydrocarbons which are recycled from the hydration stages, is dehydrogenated.

8. Process according to one of the Claims 1 to 7, characterized in that a mixture of water and sec-butyl alcohol is separated from the hydration reaction mixture by rectification, sec-butyl alcohol is extracted from this mixture by treating with a $C_4$-fraction containing isobutene, a mixture containing sec-butyl alcohol and isobutene and possibly water is distilled off from this extract and fed to the etherification, while the $C_4$-fraction freed from sec-butyl alcohol is recycled to the extraction and the aqueous phase from the extraction is recycled to the hydration.

9. Process according to one of the Claims 1 to 7, characterized in that sec-butyl alcohol is extracted from the hydration reaction mixture by treating it with a hydrocarbon mixture consisting mainly of n-butane and butene-2, from this mixture sec-butyl alcohol, possibly containing water, is distilled off and fed to the etherification, while the hydrocarbon mixture, freed of sec-butyl alcohol, is proportionally recycled to the hydration and the extraction, and the aqueous phase from the extraction is recycled to the hydration.

10. Process for producing mixtures of isopropyl tert-butyl ether and sec-butyl tert-butyl ether and methyl tert-butyl ether, which mixtures may contain isopropyl alcohol and/or sec-butyl alcohol and/or tert-butyl alcohol and/or methyl alcohol from a mixture of light hydrocarbons containing $C_1$ and $C_2$ hydrocarbons beside $C_3$ and $C_4$ hydrocarbons, according to Claim 1, characterized in that methane and ethane are separated from the hydrocarbon mixture used as raw material and are combined with a stream of methane, ethane and ethene generated during dehydrogenation of butane and propane, methyl alcohol is produced by catalytic reforming this mixture in the manner known per se with steam at a pressure of 40 to 100 bar and temperatures of 210 to 300° C, the $C_4$ hydrocarbon fraction containing isobutene which has not reacted is separated from the mixture leaving the etherification and containing isopropyl alcohol, isopropyl tert-butyl ether, sec-butyl alcohol, sec-butyl tert-butyl ether and possibly tert-butyl alcohol, this $C_4$ fraction is combined with the portion of the isobutene contained in the dehydrogenation reaction mixture and methyl alcohol and isobutene are reacted in the manner known per se to form methyl tert-butyl ether.

**Revendications**

1. Procédé pour la fabrication d'un mélange de tert.-butyléther d'isopropyle et de tert.-butyléther de butyl sec., contenant éventuellement de l'alcool isopropylique et/ou de l'alcool butylique sec., et/ou de l'alcool butylique tert., à partir d'un mélange d'hydrocarbures légers qui contient du propane et du butane, procédé caractérisé en ce que l'on sépare une fraction propane et une fraction butane, isomérise le n-butane, déshydrogénise catalytiquement le butane et le propane, hydrogénise sélectivement le butadiène avec transformation simultanée du butène-1 en butène-2, transforme le propène avec de l'eau en alcool isopropylique, à partir du mélange contenant principalement le propène et le propane, transforme une partie de l'isobutène contenu dans le mélange de la réaction de déshydrogénation, avec un mélange, contenant éventuellement de l'eau, d'alcool isopropylique et d'alcool butylique sec. recyclé en un mélange contenant du tert.-butyléther d'isopropyle et du tert.-butyléther de butyle sec., transforme le butène-2 qui n'a pas réagi du mélange d'hydrocarbures, contenant principalement du butène-2 et du butane, avec le l'eau, en alcool butylique sec. et renvoie ce dernier à l'éthérification, pendant que les hydrocarbures qui n'ont pas réagi, provenant de l'hydratation du propène et du butène-2, ainsi que l'isobutane qui n'a pas réagi, provenant de l'éthérification, sont retournés dans l'étape de déshydrogénation.

2. Procédé suivant la revendication 1, caractérisé en ce que l'on déshydrogénise la fraction propane en commun avec la fraction butane et sépare du mélange de la réaction de déshydrogénation obtenu, à nouveau, une fraction en C$_3$.

3. Procédé suivant l'une des revendications 1 et 2, caractérisé en ce que l'on fabrique, à partir d'un mélange contenant de l'alcool butylique sec. et de l'alcool isopropylique et d'isobutène liquide, en présence de catalyseurs acides, à des températures de 20 à 150°C, de préférence 30 à 60°C, un mélange contenant du tert.-butyléther d'isopropyle et du tert.-butyl éther de butyl sec., 0,5 à 10 et de préférence 1 à 3 moles d'isobutène étant mise en oeuvre par mole d'alcool.

4. Procédé suivant l'une quelconque des revendications 1 à 3, caractérisé en ce que l'on sépare un mélange d'alcool isopropylique et d'alcool butylique sec. qui n'ont pas réagi par traitement du mélange de la réaction d'éthérification avec de l'eau.

5. Procédé suivant l'une des revendications 1 ou 2, caractérisé en ce que l'on transforme un mélange d'alcool butylique sec., d'alcool isopropylique et d'eau, en présence de catalyseurs acides, avec de l'isobutène liquide, à des températures de 20 à 150°C de préférence de 30 à 80°C, en un mélange contenant de l'alcool butylique tert., du tert.-butyléther de butyle sec. et du tert.-butyléther d'isopropyle, la quantité d'isobutène mise en oeuvre étant telle que l'on trouve pour 1 mole d'eau, 1 à 20 moles d'isobutène et pour 1 mole d'alcool, 0,1 à 10 moles d'isobutène.

6. Procédé suivant l'une quelconque des revendications 1 à 5, caractérisé en ce que par rectification, on sépare, du mélange de la réaction d'hydratation, un mélange d'alcool isopropylique et d'eau, extrait de ce dernier, par traitement avec une fraction en C$_4$ contenant l'isobutène, l'alcool isopropulique, à partir duquel on reprend par distillation un mélange contenant l'alcool isopropylique et l'isobutène ainsi qu'éventuellement de l'eau et l'envoie à l'éthérification, pendant que la fraction en C$_4$ exempte d'alcool isopropylique est retournée à l'extraction, et la phase aqueuse provenant de l'extraction est retournée à l'hydratation.

7. Procédé suivant l'une des revendications 1 à 6, caractérisé en ce que l'isomérisat de la fraction butane est divisé en une fraction n-butane et une fraction isobutane, que la fraction n-butane est retournée à l'isomérisation, la fraction isobutane, y compris l'isobutane retourné après l'éthérification, et y compris les hydrocarbures retournés de l'étape d'hydratation étant soumise à une déshydratation.

8. Procédé suivant l'une quelconque des revendications 1 à 7, caractérisé en ce que l'on sépare, par rectification, du mélange de la réaction d'hydratation, un mélange d'eau et d'alcool butylique sec., sépare par extraction de ce dernier, par traitement avec une fraction en C$_4$ contenant de l'isobutène, l'alcool butylique secondaire, puis distille un mélange contenant de l'alcool butylique sec. et de l'isobutène ainsi qu'éventuellement de l'eau, et l'envoie à l'éthérification, pendant que la fraction en C$_4$ dont on a extrait l'alcool butylique sec. dans l'extraction, et la phase aqueuse provenant de l'extraction sont retournées à l'hydratation.

9. Procédé suivant l'une quelconque des revendications 1 à 7, caractérisé en ce que l'on extrait du mélange réactionnel de l'hydratation, par traitement avec un mélange constitué principalement de n-butane et de butène-2, de l'alcool butylique sec., en retire par distillation l'alcool butylique sec. contenant éventuellement de l'eau, et l'envoie à l'éthérification, pendant que le mélange d'hydrocarbures exempt d'alcool butylique sec. est enoyé par parties à l'hydratation et à l'extraction, la phase aqueuse provenant de l'extraction étant retournée à l'hydratation.

10. Procédé pour la fabrication de mélanges contenant éventuellement de l'alcool isopropylique et/ou de l'alcool butylique sec., et/ou de l'alcool butylique tert., et/ou des mélanges, contenant de l'alcool méthylique, de tert.-butyléther d'isopropyle et de tert.-butyl-éther de butyle sec., et de tert.-butyléther de méthyle, à partir d'un mélange d'hydrocarbures légers qui contient, à côté d'hydrocarbures en C$_3$ et C$_4$, aussi des hydrocarbures en C$_1$ et C$_4$, suivant la revendication 1, caractérisé en ce que l'on sépare le méthane et l'éthane du mélange d'hydrocarbures exploité comme matière première, et les rassemble avec le courant de méthane, éthane et éthène, obtenu lors de la déshydrogénation du butane et du propane, fabrique, par reformage de ce mélange avec de la vapeur d'eau d'eau, d'une façon connue en soi, par catalyse sous une pression de 40 à 100 bars et à des températures de 210 à 300°C, de l'alcool méthylique, que l'on sépare du mélange d'éthérification contenant de l'alcool isopropylique, du tert.-butyléther d'isopropyle, de l'alcool butylique sec., du tert.-butyléther de butyle sec. et éventuellement de l'alcool butylique tert., la fraction d'hydrocarbures en C$_4$ contenant de l'isobutène qui n'a pas réagi, la réunit avec une partie de l'isobutène contenu dans le mélange de déshydrogénation, et fabrique, à partir de l'alcool méthylique et de l'isobutène, d'une façon connue en soi du tert.-butyléther de méthyle.